Europäisches Patentamt

(19) European Patent Office

Office européen des brevets

(11) Publication number: **0 050 514**
**B1**

(12) **EUROPEAN PATENT SPECIFICATION**

(45) Date of publication of patent specification: **18.07.84**

(51) Int. Cl.³: **A 61 L 15/01,**
**A 61 L 15/06, A 61 F 13/00**

(21) Application number: **81304884.0**

(22) Date of filing: **20.10.81**

(54) **Wound dressings and processes for their preparation.**

(30) Priority: **22.10.80 GB 8034085**

(43) Date of publication of application:
**28.04.82 Bulletin 82/17**

(45) Publication of the grant of the patent:
**18.07.84 Bulletin 84/29**

(84) Designated Contracting States:
**AT BE CH DE FR GB IT LI NL SE**

(56) References cited:
**EP - A - 0 006 263**
**GB - A - 1 142 323**
**GB - A - 1 144 176**
**GB - A - 1 527 592**
**US - A - 4 133 310**

(73) Proprietor: **Smith and Nephew Associated Companies p.l.c.**
**2, Temple Place Victoria Embankment London WC2R 3BP (GB)**

(72) Inventor: **Lloyd, Ronald**
**63 Cambridge Road**
**Sawbridgeworth Hertfordshire (GB)**

(74) Representative: **Cole, William Gwyn**
**Corporate Patents Department Smith and Nephew Research Limited Gilston Park Harlow Essex CM20 2RQ (GB)**

(56) References cited:
**ULLMANN'S ENCYKLOPÄDIE DER TECHNISCHEN CHEMIE, Vol. 19, 4th Ed., 1980, pages 330-332**
**Seymour et al., Journal of the Plastics Institute, Conference Supplement No. 3, 1968, pages 117-118**

The file contains technical information submitted after the application was filed and not included in this specification

Courier Press, Leamington Spa, England.

## Description

The present invention relates to wound dressings which comprise a wound facing layer of a polyurethane net and to their manufacture.

The use of integral polymer nets made of a polyolefin such as high density polyethylene and polypropylene as wound facing materials for absorbent pads has been disclosed in British Patent Specification No. 1,142,323. The wound facing materials of these dressings are relatively non-conformable. It would be desirable to have available more conformable wound facing substrates. It is also desirable that the components of a wound dressing, in particular the wound facing substrate, should be sterile.

Certain polyurethane nets are disclosed in US Patent No. 3,913,510 as carpet backing materials which hold carpet tufts in position without the use of an adhesive. However there is no suggestion that such net can be used for other purposes such as wound dressings. The skilled worker will appreciate that it is not normal to obtain materials from the carpeting art to use as wound dressings. GB—A—1527592 discloses mats of electrostatically spun fibres of polyurethane with fibre diameters of 0.1 to 25 $\mu$m which can be used as a wound facing on an absorbent layer. Such fine fibre mats are not integral nets. Furthermore GB—A—1527592 discloses that these polyurethane fibre mat wound facings are wound adherent. EP—A—6263 discloses an absorbent rayon web which is stabilised by melting into the web an open thermoplastic polymer web of polyurethane. However, there is no suggestion in EP—A—6263 to an integral net of polyurethane which is suitable for contacting wounds. GB—A—1,144,176 discloses a process of making a fine pattern in relief on a polyurethane sheet by casting a polyurethane reaction mixture on a patterned silicone elastomer belt and heating the mixture to cure the polyurethane. However GB—A—1,144,176 does not disclose a process of making a polyurethane net nor does it disclose or suggest that the cast polyurethane sheet is suitable for contacting wounds. However it has now been discovered that nets of elastomeric polyurethane can be employed to provide a highly conformable sterile wound facing. It has also been found that such nets can be sterilised so that wound dressings employing them can be provided in sterile form.

The present invention provides a sterile wound dressing which is contained within a bacteria-proof package and which comprises a wound facing layer and optionally an absorbent layer which wound facing layer comprises a sterile net comprising intersecting strands in which the strands and junctures are formed integrally characterised in that the net comprises elastomeric polyurethane and has about 4 to 40 intersections per cm of strand which strands define openings having a minimum dimension of 0.05 mm and maximum dimension of 2 mm.

The net will be an integral net (that is a net in which the strands and junctures are formed integrally, for example during manufacture).

Since the net is intended for use in contact with lesions, the elastomeric polyurethane employed will be non toxic.

It is desirable that the polyurethane employed in this invention has a sufficient moisture vapour transmission rate to allow moisture from the wound area to transmit through the integral net material as well as through the interstices. Suitable polyurethanes will thus have a moisture transmission rate of at least 600 g/m² and preferably at least 1000/m²/24 h at 37.5°C at 100% to 10% relative humidity difference in the form of a continuous film 25 $\mu$m thick.

Particularly suitable elastomeric polyurethanes are linear polyurethanes for example those containing polyether or polyester groups. Certain apt elastomeric linear polyester polyurethanes are disclosed in U.S. Patent Specification No. 2,871,218. Certain apt elastomeric linear polyether polyurethanes are disclosed in U.S. Patent Specification No. 2,899,511. It is preferred to employ polyether polyurethanes and especially linear polyether polyurethanes.

Favoured elastomeric linear polyurethanes include Estanes (registered trade mark) from B. F. Goodrich.

Preferred polyurethanes include Estane 5714 F1, 5702 and 5703.

Suitable elastomeric polyurethanes have an elongation at break of 300% to 800% and preferably of 500% to 700% when tested according to ASTM D882 Estane 5714 F1 is an elastomeric linear polyether polyurethane which has an elongation break of 560% when tested according to ASTM D882. A 25 $\mu$m thick film of Estane 5714 F1 has a moisture vapour transmission rate of approximately 1800 g/m²/24 h at 37°C at 100% to 10% relative humidity difference. Estanes 5702 and 5703 are polyester polyurethanes which have an elongation at break of 700% when tested according to ASTM D882. A 25 $\mu$m thick film of Estane 5702 has a moisture vapour transmission rate of approximately 1600 g/m²/24 h at 37°C at 100% to 10% relative humidity difference.

The elastomeric polyurethane can contain a filler as long as the filler employed does not adversely effect the elastomeric properties of the polyurethane. Suitable fillers can give the integral polyurethane net and wound dressing of the invention a desirable white textile appearance. The elastomeric polyurethane can contain up to 50 parts by weight (but more suitably not more than 20 parts by weight) of titanium dioxide filler.

The net of elastomeric polyurethane of the wound dressing of the invention can have any convenient form depending on the chosen arrangement of strand, juncture and hole areas and also their shapes and relative size.

In one preferred form the net consists essentially of longitudinal and transverse strands intersecting at right angles to give a square grid hole pattern.

Suitable nets of this type aptly have 4 to 40 strands per cm and desirably 8 to 40 strands per cm and preferably 8 to 24 strands per cm in both longitudinal and transverse directions.

Fine integral elastomeric polyurethane nets of 8 to 40 strand counts have not been previously known but are now believed to be particularly suitable for use in contacting wound. Thus in a further aspect the sterile wound dressing of the invention comprises a sterile integral net of elastomeric polyurethane which has a strand count of 8 to 40 per cm. Preferably such a net has a strand count of 8 to 24 per cm.

Such nets are desirable since when used as a wound facing for example for an absorbent dressing the dimensions of the openings of the integral net are small enough to prevent the absorbent layer, especially when made of absorbent fibres, from protruding through the hole thus causing irritation of the wound.

Variations on the square grid pattern can give other desirable forms of the integral net. Unequal density of strands in either the longitudinal or transverse directions will give rectangular hole areas. Continuous parallel strands in one direction with a staggered arrangement of connecting strands in the other direction will give a "brickwork" pattern. The strands in one direction may be thicker than those in the other direction. Such variation may be employed to produce nets which have different mechanical properties in the longitudinal or transverse direction.

Other preferred forms of the nets can have strands at an angle to the longitudinal or transverse direction i.e. diagonal strands. Another preferred form of the integral polymer net can have a staggered arrangement of circular or approximately circular (e.g. hexagonal) arrangements of strands and hole areas. The net can be in the form of a mixed pattern of two or more of the arrangements.

Most suitably integral elastomeric polyurethane nets for wound dressings of the invention have a plurality of openings with a dimension from 0.05 mm to 1 mm, desirably from 0.1 to 0.5 mm.

Suitable nets have a thickness of 25 $\mu$m to 250 $\mu$m, and preferably 50 $\mu$m to 150 $\mu$m. Similarly suitable nets can have a weight per square metre of 10 g to 80 g, and preferably 15 g to 50 g.

Suitable integral nets of elastomeric polyurethane have an elongation at break of 100% to 800%, desirably 200% to 750% and preferably 300% to 700% when measured as a 2.5 cm wide strip at a 30 cm/min strain rate at 20°C.

The integral net of elastomeric polyurethane should be capable of being sterilised independently or in combination with the other components of the wound dressing. Suitable methods of sterilising the net or a wound dressing containing the net include steam autoclaving, ethylene oxide gas, electron beam irradiation and gamma irradiation.

The absorbent layer of wound dressings of the invention can be selected from absorbent materials used in conventional wound dressings. Suitable absorbent materials include knitted, woven and nonwoven fabrics containing cellulosic fibres, synthetic fibres or mixtures thereof.

A favoured absorbent layer consists essentially of a thin layer of staple fibres containing 80% by weight of cotton fibres and 20% by weight of acrylic fibres lightly bonded and laid on an open weave cotton gauze. The absorbent layer may have a thickness of approximately 6 mm and a weight per unit area of approximately 200 g/m².

To enable the wound dressing to function adequately the integral elastomeric polyurethane nets should be in contact with and preferably attached to or about the wound facing side of the absorbent layer.

The net can be folded around and attached to the non-wound contacting side of the absorbent layer but it is preferred that the net is substantially coextensive with the absorbent layer and attached over the whole of the wound facing surface. Suitable methods of attaching the nets to the absorbent layer include heat sealing, adhesive bonding or forming the absorbent layer or net in situ whilst in contact with the other layer. Preferably a layer of absorbent material is bonded to an integral net of elastomeric polyurethane to form a composite strip which is then cut into suitable size wound dressings.

The wound dressings of the invention can be in various conventional forms. A preferred wound dressing is in the form of a square or rectangular strip. Convenient sizes of the strips for treating wounds are 5 cm×5 cm, 10 cm×10 cm and 20 cm×20 cm. Wound dressings of the invention can have a thickness of 1 mm to 20 mm.

Another preferred wound dressing is in the form of an adhesive dressing in which the absorbent layer in contact with the integral net of elastomeric polyurethane constitutes the absorbent pad of the dressing.

The wound dressing of the invention can be sealed in a bacteria-proof package and the package sterilised by conventional methods such as ethylene oxide gas, steam autoclaving, electron irradiation and gamma irradiation.

The invention provides a method of making a sterile integral net suitable for use in a sterile wound dressing of the invention which comprises casting an elastomeric polyurethane as a solution, dispersion, hot melt or powder onto a surface having a pattern of discrete raised areas and interconnected recessed areas which correspond to the openings and strands of a sterile integral net of the invention and heating, drying or cooling the cast net as is appropriate to form a solid net.

A suitable method of forming the integral net of polyurethane used in the invention is a method in which an elastomeric polyurethane in a flowable state is cast onto a surface having a pattern of discrete raised areas and inter connected recessed areas, and treated to form a solid integral net. The flowable state of the elastomeric polyurethane can include solutions, dispersions, hot melts and powders.

The term "treated" as used in the method of making the integral polymer nets used in the invention means the physical and/or chemical process which can convert the cast polymer net from a flowable form to a solid form. If more than one process is involved then the process can be carried out simultaneously or consecutively as is convenient.

In the process of the invention the elastomeric polyurethane can be cast in a molten state for example by hot melt extrusion. However it is preferred that the elastomeric polyurethane is cast as a solution. Thus the polyurethane employed in this invention is preferably a linear polyurethane.

Suitable casting solutions may contain 15% to 35%, and preferably 20 to 30% by weight of elastomeric polyurethane. A favoured casting solution contains 20 to 25% by weight of Estane 5702 or Estane 5703 in acetone. Another favoured solution contains 25% to 30% of Estane 5714F in tetrahydrofuran or mixtures of tetrahydrofuran and acetone.

Conveniently the cast elastomeric polyurethane is treated by passage through a hot oven in order to evaporate the solvent and form a solid integral net. However other treatments are also possible. These treatments include drying a liquid dispersion, cooling a molten polymer and crosslinking a polymer in a liquid or solid form.

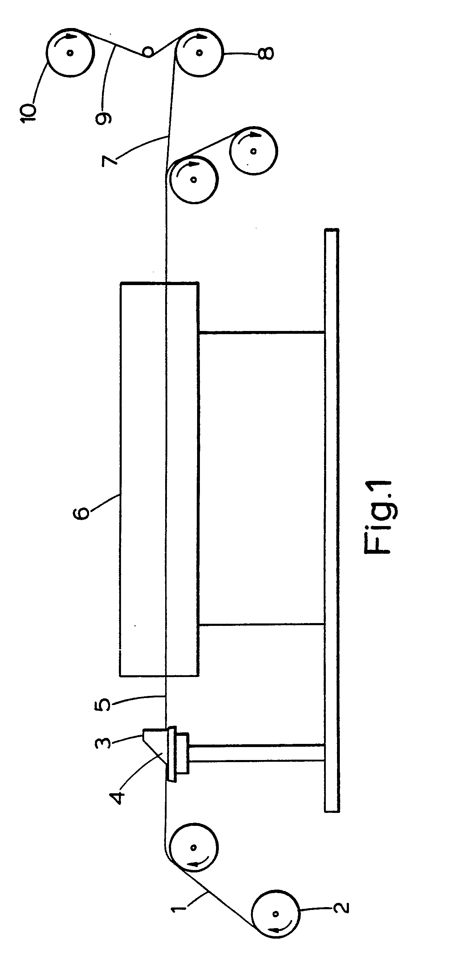

Figure 1 illustrates a process of making the nets used in the invention. A thermoplastic film (1) with an embossed pattern on its upper surface fed from roll (2) to coating head (3) where a solution (4) cast onto the recesses of the embossed sheet. The wet cast net (5) on the embossed sheet passed into an oven (6) where it is dried. The dried cast net (7) then separated from the embossed sheet (1) and wound up onto roller (8) where it is also interleaved with a release paper (9) fed in from roll (10).

Figure 2 is a perspective view of a casting process used to form the nets used in the invention.

Figure 3 is a side view of the casting head and casting process of Figure 2 with the side plates removed.

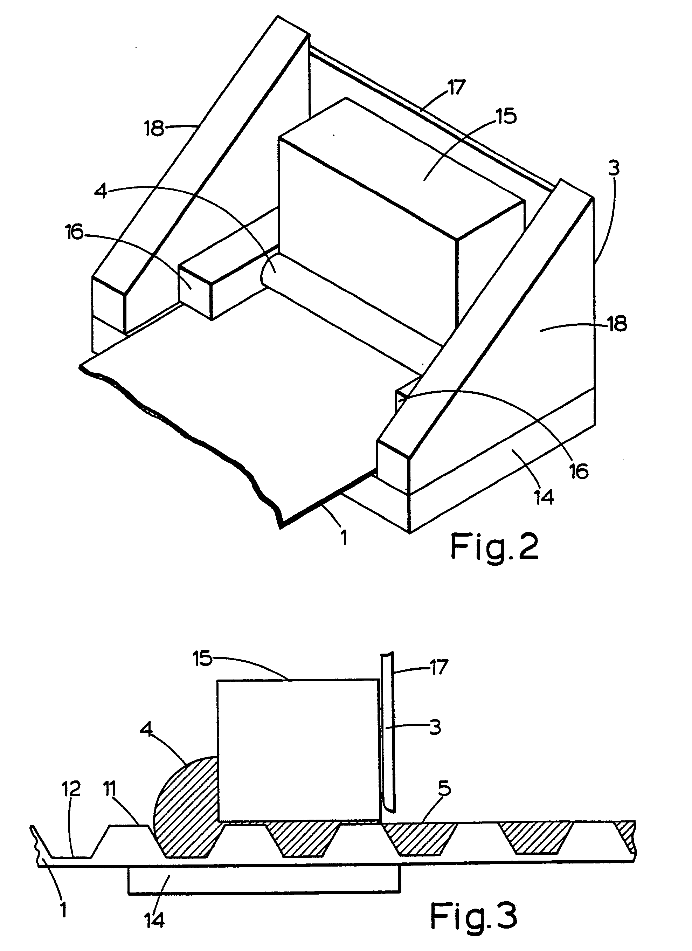

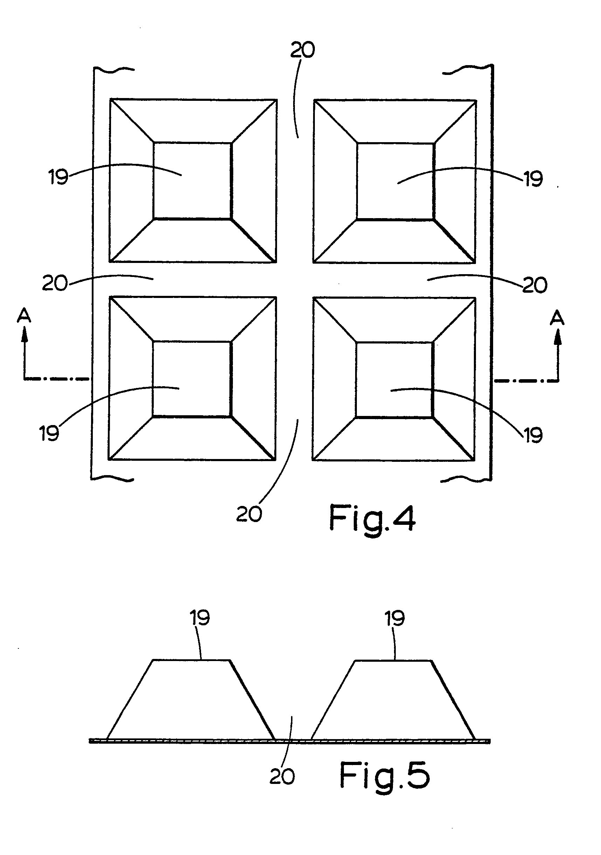

In Figure 2 and Figure 3 the embossed film (1) having a pattern on its surface of discrete raised areas (11) and interconnected recesses (12) coated with a polymer solution (4) by means of a coating head (3) to form a cast net (5). The coating head (3) has a flat bed (14), a spreading block (15) and side spreading guides (16) which are next to the adjustable blade (17) fixed to the sides of the coating head (18).

Most of the spreading head components can be made from metal in conventional manner but in order to provide less friction against the film it is advantageous to make the spreading block and the guides or their film contacting surfaces from a fluorocarbon polymer such as polytetrafluoroethylene. It is preferred that the base of the spreading block should be large enough to span the discrete raised areas of the embossed film in order to prevent the block catching in the recessed areas. The spreading block and adjustable blade can be replaced by a single broad based adjustable spreading device. Alternatively coating heads using fixed or rotating roller doctor devices can be used.

Figure 4 illustrates a plan view of an embossed pattern sheet suitable for forming a net used in invention.

Figure 5 is a cross section through line A—A of Figure 4.

Figure 4 shows discrete raised areas (19) arranged in a square pattern to give a square grid pattern of recesses (20).

In Figure 5 a section through line A—A of Figure 4 shows the discrete raised areas (19) in the shape of truncated square pyramids and conical recesses (20).

A particularly preferred embossed pattern sheet has 8 per cm raised areas in the form of square truncated pyramids 1 mm wide and 0.5 mm high with sides sloping to a 60° conical angle and a corresponding longitudinal and transverse square grid recess 0.25 mm wide at the base and 0.75 mm at the top.

Other suitable embossed sheet patterns include those which have raised areas of a rectangular, diamond, circular or approximately circular (e.g. hexagonal) shape so as to give the corresponding shape to the hole area in the integral net. Similarly the recessed areas in one direction can be of different depth, different density or direction, or can be diagonal to the longitudinal direction to give the corresponding shape or arrangement to the strand and/or juncture areas of the integral polymer net. Mixtures of two or more of these patterns can also be used.

The casting surface may be in the form of a roller, an endless flexible belt or a length of flexible sheet material. It is preferred that the casting surface has release properties in order to enable the dried net to be removed from the casting surface. A preferred casting surface is a melt embossed sheet of a polyolefin polymer made by the method given in British Patent Specification No. 1055963.

Example 1

A solution containing 30% by weight of Estane 5714 F1 in tetrahydrofuran was cast into the recesses of a 15 cm wide melt embossed high density polyethylene sheet by means of the blade over flat bed spreading technique. The sheet had a melt embossed pattern of 8 per cm raised areas in the form of square truncated pyramids 1 mm wide at their base and 0.5 mm high with sides sloping to a solid conical angle of 60°. The wet cast net on the embossed film was dried by passage through a hot air circulating oven at a temperature of 90°C to 100°C for two minutes. The dried cast net was separated from the embossed film and wound onto a roller interleaved with a double-sided silicone release coated paper.

The resultant cast integral elastomeric polyurethane net had the following properties: Weight (per m²) 40 g; thickness 100 $\mu$m to 125 $\mu$m hole size 0.3 to 0.4 mm; tensile strength (g/2.5 cm wide), machine direction 800$\pm$51, transverse direction 664$\pm$57; elongation at break (%), machine direction 389$\pm$24, transverse direction 374$\pm$24.

The net was bonded to an absorbent layer weighing approximately 200 g consisting of 80% cotton fibres and 20% acrylic fibres lightly bonded by an acrylic latex on a woven gauze base, by passage through the nip of pressure rollers heated to 100°C. The resultant strip cut into 5 cm×5 cm dressings and packed into steam autoclavable pouches formed by heat sealing a 59 g/m² laquered Kraft paper from Bemrose Flexible Packaging Ltd. The dressings were sterilised by steam autoclaving at 115°C for 30 minutes.

Example 2

A solution containing 20% by weight of Estane 5702 in acetone was cast into the recesses of a 15 cm wide high density polyethylene embossed sheet and dried in the same manner as Example 1. The resultant cast net had the following properties: Weight (per m²) 20 to 25 g; thickness 50 $\mu$m; hole size 0.3 to 0.4 mm.

The net was laminated to an absorbent layer and made into sterile dressings as Example 1.

Example 3

A solution containing 20% by weight of Estane 5714 F1 in tetrahydrofuran was cast into the recesses of a 15 cm wide melt embossed high density polyethylene sheet by means of the blade over flat bed technique. The sheet had a melt embossed pattern of 20 per cm raised areas in the form of square pyramids 0.4 mm wide at their base, 0.3 mm high and side sloping to a solid angle of 80°C, arranged in staggered rows in the length of the sheet and parallel rows across the sheet i.e. in the form of a brick pattern. The wet cast net was dried in the same manner as Example 1.

The resultant cast net had the following properties: Weight (per m²) 14 g; thickness 50 $\mu$m; hole size 0.1 to 0.2 mm; tensile strength (g/2.5 cm wide), machine direction 488$\pm$73, transverse direction 493$\pm$74; elongation at break (%) machine direction 408$\pm$53, transverse direction 428$\pm$52.

The net was laminated on an absorbent layer and made into sterile dressings of Example 1.

Example 4

A solution containing 20% by weight of TiO$_2$ filled Estane 5714 F1 (contains 15 p.h.r. of TiO$_2$) in tetrahydrofuran was cast into a sheet and formed into a net as in Example 3.

The resultant cast net had the following properties: Weight (per m²) 19 g; thickness 50 $\mu$m; hole size 0.1 to 0.2 mm. tensile strength (g/2.5 cm wide), machine direction 313$\pm$33; transverse direction 312$\pm$26; elongation at break (%) Machine direction 307$\pm$44, transverse direction 364$\pm$44.

The net was laminated to an absorbent layer and made into sterile dressings as in Example 1.

Example 5

The net of Example 4 was cut into 5 cm×5 cm squares and packed and sterilised as the wound dressing of Example 1 to give a sterile net of elastomeric polyurethane.

Example 6

A solution containing 20% by weight of Estane 5714 F1 in tetrahydrofuran was cast into the recesses of a 30 cm wide melt embossed high density polyethylene sheet by means of a blade over flat bed coating technique. The sheet had a melt embossed pattern of 6 per cm raised areas arranged in a diamond pattern of rows at an angle of 45° to the longitudinal direction of the sheet in the form of square truncated pyramids 1.3 mm wide at the base and 0.5 mm high with side sloping to a solid conical angle of 70°. The wet cast net on the embossed film was dried by passage through a hot air oven at a temperature of 70°C to 80°C for 1 minute. The dried case net on the embossed film was wound onto a roller. The resultant cast integral elastomeric polyurethane net had the following properties: Weight (per square metre) 37g; tensile strength (g/2.5 cm wide) machine direction 536$\pm$33, transverse direction 526$\pm$39; elongation at break (%) machine direction 309$\pm$18, transverse direction 307$\pm$53.

Individual 30 cm×40 cm pieces of the cast net were placed between release paper sheets, packed into sealed bacteria proof autoclave sterilising bags (available from J. Dickinson & Co.) and sterilised by exposure to gamma irradiation, electron irradiation at 2.5 Mrads or ethylene oxide gas.

Samples of the sterilised net were tested for sterility by incubation in a broth for 7 days at a temperature of 30°C. The lack of any bacterial growth indicated that the nets were sterile.

The tensile strengths and elongations at break of the sterilised nets were not significantly different from that of the nets before sterilisation.

## Claims

1. A sterile wound dressing which is contained within a bacteria-proof package and which comprises a wound facing layer and optionally an absorbent layer which wound facing layer comprises a sterile net comprising intersecting strands in which the strands and junctures are formed integrally, characterised in that the net comprises elastomeric polyurethane and has about 4 to 40 intersections per cm of strand which strands define openings having a minimum dimension of 0.05 mm and a maximum dimension of 2 mm.

2. A sterile wound dressing as claimed in claim 1 in which the net has 4 intersections per cm of strand.

3. A sterile wound dressing as claimed in either of claims 1 or 2 in which the elastomeric polyurethane is a linear polyurethane having an elongation at break of 300% to 800%.

4. A sterile wound dressing as claimed in claim 3 in which the linear polyurethane comprises a linear polyether polyurethane.

5. A sterile wound dressing as claimed in any of claims 1 to 4 in which the net has longitudinal and transverse strands which intersect at right angles to give a square pattern net.

6. A sterile wound dressing as claimed in any of claims 1 to 5 in which the net has intersecting diagonal strands which form a diamond pattern net.

7. A sterile wound dressing as claimed in any of claims 1 to 6 in which the net has a weight per unit area of 15 g/m² to 50 g/m².

8. A sterile wound dressing as claimed in any of claims 1 to 7 in which the net has an elongation at break of 200% to 750% when measured as a 2.5 cm wide strip at 30 cm/min strain rate at 20°C.

9. A sterile wound dressing as claimed in any of claims 1 to 8 which comprises an absorbent layer characterised in that the net is substantially coextensive with the absorbent layer and is attached over the whole of the wound facing surface.

10. A method of making a sterile net suitable for use in a sterile wound dressing as claimed in any of claims 1 to 9 which method comprises casting an elastomeric polyurethane as a solution, dispersion, hot melt or powder onto a surface having a pattern of discrete raised areas and interconnected recessed areas which correspond to the openings and strands of a sterile net as defined in claim 1 and heating, drying or cooling the cast net as appropriate to form a solid net.

11. A method of making a sterile net as claimed in claim 10 in which the elastomeric polyurethane is cast in solution on to an embossed surface.

12. A method of making a sterile net as claimed in either of claims 10 or 11 in which the casting surface has a square pattern of discrete raised areas in the shape of truncated pyramids and a corresponding square grid pattern of interconnected recesses.

13. A method of making a sterile net as claimed in any of claims 10 to 12 which comprises casting a solution containing 15% to 35% by weight of elastomeric polyurethane.

14. A method of making a sterile integral net as claimed in claim 13 in which the solution contains 20% to 30% by weight of elastomeric polyurethane.

15. A method of making a sterile wound dressing as claimed in any of claims 1 to 9 which comprises sterilising the wound dressing contained with a bacteria-proof package by treatment with ethylene oxide gas, electron beam irradiation or gamma irradiation.

## Patentansprüche

1. Steriler Wundverband, der in einer bakteriensicheren Packung enthalten ist und eine wundseitige Schicht und gegebenenfalls eine absorbierende Schicht umfaßt, wobei die wundseitige Schicht ein steriles Netz mit sich schneidenden Fäden umfaßt, worin die Fäden und Verbindungen integral ausgebildet sind, dadurch gekennzeichnet, daß das Netz elastomeres Polyurethan umfaßt und etwa 4 bis 40 Schnittstellen pro cm Faden hat, wobei die Fäden Öffnungen mit einer Mindestabmessung von 0,05 mm und einer Maximalabmessung von 2 mm haben.

2. Steriler Wundverband nach Anspruch 1, worin das Netz 4 Schnittstellen pro cm Faden hat.

3. Steriler Wundverband nach jedem der Ansprüche 1 oder 2, worin das elastomere Polyurethan ein lineares Polyurethan mit einer Bruchdehnung von 300 % bis 800 % ist.

4. Steriler Wundverband nach Anspruch 3, worin das lineare Polyurethan ein lineares Polyetherpolyurethan umfaßt.

5. Sterilier Wundverband nach irgend einem der Ansprüche 1 bis 4, worin das Netz Längs- und Querfäden hat, die sich rechtwinklig kreuzen, um ein Netz mit quadratischem Muster zu ergeben.

6. Steriler Wundverband nach irgend einem der Ansprüche 1 bis 5, worin das Netz schneidende Diagonalfäden hat, die ein Netz mit Diamantmuster bilden.

7. Steriler Wundverband nach irgend einem der Ansprüche 1 bis 6, worin das Netz ein Gewicht pro Einheitsfläche von 15 g/m² bis 50 g/m² hat.

8. Steriler Wundverband nach irgend einem der Ansprüche 1 bis 7, worin das Netz eine Bruchdehnung von 200 % bis 750 % hat, gemessen als 2,5 cm breiter Streifen bei einer Zugspannungsrate von 30 cm/min bei 20°C.

9. Steriler Wundverband nach irgend einem der Ansprüche 1 bis 8, der eine absorbierende Schicht umfaßt, dadurch gekennzeichnet, daß das Netz mie der absorbierenden Schicht im wesentlichen gemeinsam dehnbar ist und über der ganzen wundseitigen Oberfläche angebracht ist.

10. Verfahren zur Herstellung eines sterilen Netzes, geeignet zur Verwendung in einem sterilen Wundverband, wie in irgend einem der Ansprüche 1 bis 8 beansprucht, welches das Gießen eines elastomeren Polyurethans als Lösung, Dispersion, heiße Schmelze oder Pulver auf eine Oberfläche mit einem Muster diskreter erhobener Bereiche und untereinander verbundener, ausgenommener Bereiche, die den Öffnungen und Fägen eines sterilen Netzes, wie in Anspruch 1 definiert, entsprechen, und das Erwärmen, Trocknen oder Kühlen des gegossenen Netzes, je nach Eignung zur Bildung eines festen Netzes, umfaßt.

11. Verfahren zur Herstellung eines sterilen Netzes nach Anspruch 10, worin das elastomere Polyurethan in Lösung auf eine geprägte Oberfläche gegossen wird.

12. Verfahren zur Herstellung eines sterilen Netzes nach jedem der Ansprüche 10 oder 11, worin die Gießoberfläche ein quadratisches Muster diskreter erhöhter Bereiche in Form kegelstumpfförmiger Pyramiden und ein entsprechendes quadratisches Gittermuster untereinander verbundener Ausnehmungen aufweist.

13. Verfahren zur Herstellung eines sterilen Netzes nach irgend einem der Ansprüche 10 bis 12, welches das Gießen einer Lösung, die 15 bis 35 Gew.-% elastomeren Polyurethans enthält, umfaßt.

14. Verfahren zur Herstellung eines sterilen integralen Netzes nach Anspruch 13, worin die Lösung 20 bis 30 Gew.-% elastomeren Polyurethans enthält.

15. Verfahren zur Herstellung eines sterilen Wundverbandes nach irgend einem der Ansprüche 1 bis 9, bei dem der in einer bakteriensicheren Packung enthaltene Wundverband durch Behandeln mit Ethylenoxidgas, Bestrahlen mit Elektronenstrahl oder $\gamma$-Bestrahlung sterilisiert wird.

## Revendications

1. Pansement stérile contenu dans une enveloppe étanche aux bactéries et comportant une couche dirigée vers la plaie et, facultativement une couche absorbante, cette couche dirigée vers la plaie étant constituée par un tulle ou filet stérile comportant des fils ou brins s'intersectionnant dans lequel les fils ou brins et les intersections sont solidaires, caractérisé en ce que le tulle ou filet est constitué par un polyuréthane élastomère et comporte environ de 4 à 40 intersections par cm de fil, ces fils délimitant des orifices ayant une dimension minimum de 0,05 mm et une dimension maximum de 2 mm.

2. Pansement stérile suivant la revendication 1, caractérisé en ce que le tulle ou filet comporte 4 intersections par cm de fil.

3. Pansement stérile suivant la revendication 1 ou 2, caractérisé en ce que le polyuréthane élastomère est un polyuréthane linéaire ayant un allongement à la rupture de 300 % à 800 %.

4. Pansement stérile suivant la revendication 3, caractérisé en ce que le polyuréthane linéaire est un polyuréthane polyéther linéaire.

5. Pansement stérile suivant l'une quelconque des revendications 1 à 4, caractérisé en ce que le tulle ou filet comporte des fils ou brins longitudinaux et transversaux qui s'intersectionnent à angles droits pour former un tulle ou filet à trame carrée.

6. Pansement stérile suivant l'une quelconque des revendications 1 à 5, caractérisé en ce que le tulle ou filet comporte des fils ou brins s'intersectionnant en diagonale pour former un tulle ou filet à trame en losange.

7. Pansement stérile suivant l'une quelconque des revendications 1 à 6, caractérisé en ce que le tulle ou filet a un poids par unité de surface de 15 g/m² à 50 g/m².

8. Pansement stérile suivant l'une quelconque des revendications 1 à 7, caractérisé en ce que le tulle ou filet présente un allongement à la rupture de 200 % à 750 % lorsqu'il est mesuré sur une bande d'une largeur de 2,5 cm avec un taux de contrainte de 30 cm/min à 20°C.

9. Pansement stérile suivant l'une quelconque des revendications 1 à 8, comprenant une couche absorbante, caractérisé en ce que le tulle ou filet a sensiblement la même superficie que la couche absorbante et est fixé sur la totalité de la surface dirigée vers la plaie.

10. Procédé pour la fabrication d'un tulle ou filet stérile convenant à une utilisation dans un pansement stérile suivant l'une quelconque des revendications 1 à 8, ce procédé consistant à couler un polyuréthane élastomère en solution, dispersion, masse fondue à chaud ou poudre sur une surface munie d'une trame formée de petites zones en relief et de zones formant dépressions reliées entre elles, correspondant aux orifices et aux fils ou brins d'un tulle ou filet stérile tel que défini dans la revendication 1, et à chauffer, sécher ou refroidir le tulle ou filet ainsi coulé selon l'opération appropriée pour former un tulle ou filet solide.

11. Procédé pour la fabrication d'un tulle ou filet stérile suivant la revendication 10, caractérisé en ce que le polyuréthane élastomère est coulé en solution sur une surface gaufrée.

12. Procédé de fabrication d'un tulle ou filet stérile suivant la revendication 10 ou 11, caractérisé en ce que la surface de coulée présente une trame carrée formée de petites zones en relief sous la forme de pyramides tronquées et une trame en forme de grille carrée correspondante formée de dépressions reliées entre elles.

13. Procédé de fabrication d'un tulle ou filet stérile suivant l'une quelconque des revendications 10 à 12, caractérisé en ce qu'on coule une solution contenant de 15 % à 35 % en poids de polyuréthane élastomère.

14. Procédé de fabrication d'un tulle ou filet stérile d'un seul tenant suivant la revendication 13, caractérisé en ce que la solution contient de 20 % à 30 % en poids de polyuréthane élastomère.

15. Procédé de fabrication d'un pansement stérile suivant l'une quelconque des revendications 1 à 9, caractérisé en ce qu'on stérilise le pansement contenu dans une enveloppe étanche aux bactéries par traitement avec de l'oxyde d'éthylène gazeux, par irradiation par un faisceau d'électrons ou par irradiation gamma.

8

Fig.1

Fig.2

Fig.3

Fig.4

Fig.5